# EUROPEAN PATENT SPECIFICATION

(11) **EP 4 380 539 B1**
(45) Date of publication and mention of the grant of the patent: **09.09.2026**
(21) Application number: 22754875.7
(22) Date of filing: 26.07.2022
(51) Int. Cl.: A61K 8/04, A61K 8/27, A61K 8/34, A61K 8/36, A61Q 15/00

(54) **DEODORANT COMPOSITIONS**
DESODORIERENDE ZUSAMMENSETZUNGEN
COMPOSITIONS DÉODORANTES

(30) Priority: 04.08.2021 EP 21189670
(43) Date of publication of application: 12.06.2024
(73) Proprietor: Unilever IP Holdings B.V., 6708 WH Wageningen (NL); Unilever Global IP Limited, Wirral, Merseyside CH62 4ZD (GB)
(72) Inventor: HOLLMERUS, Sophie, Louise, 6708 WH Wageningen (NL); MCWALTER, Joy, Rachel, 6708 WH Wageningen (NL); ROWLAND, Alan, Philip, 6708 WH Wageningen (NL); TORRES, Ophelie, Marie-Pierre, Cecile, 6708 WH Wageningen (NL); WEDDELL, Iain, Andrew, 6708 WH Wageningen (NL)
(74) Representative: Unilever Patent Group
(86) International application number: PCT/EP2022/070879
(87) International publication number: WO 2023/011971

(56) References cited:
- WO-A1-2018/087148
- US-A1- 2009 092 568
- US-A1- 2018 207 068

## Description

### Field of Invention

The present invention is in the field of deodorant formulations, in particular antimicrobial deodorant compositions for topical application to the human body.

### Background

Antimicrobial deodorant compositions have been used to reduce body malodour by topical application to the skin of the human body for many years.

A wide range of deodorant agents have been discovered and used over the decades, including selected zinc salts.

Zinc oxide, particularly nanoparticulate zine oxide, has been used in antibacterial treatments [Y. Xie, et al, Applied Environmental Microbiology, 77 (2011), 2325], including use in food preservation [R. G. S. Chandra et al, Int. J. Sci. Research, 01(44) (2012), 524]. The latter reference also discloses the antimicrobial activity of zinc acetate and zinc hydroxyacetate.

US 4,565,693 (Marshner, 1986) discloses the use of zinc glycinate as a deodorant active material having the dual function of chemically neutralizing body odours and inhibiting bacterial growth, particularly of gram negative bacteria.

US 3,325,367 (Gillette Co., 1967) discloses zinc sulfamate and zinc phenol sulfamate as antimicrobial stringent metal salts useful in antiperspirant compositions.

H. Kuhn et al, J. Surfact. Deterg. 3 (2000), 335-343, discloses the deodorancy action of zinc ricinoleate.

WO 18/087147 and WO 18/087148 (Givaudan, 2018) disclose cosmetic compositions comprising zinc neodecanoate as a deodorant active.

### Summary of Invention

It is an object of the present invention to provide a highly effective deodorant compositions comprising selected zinc salts an antimicrobial agent.

It is a further object of the present invention to provide stable deodorant compositions comprising selected zinc salts an antimicrobial agent.

In a first aspect of the present invention, there is provided a deodorant composition which is an ethanolic solution comprising a zinc dicarboxylate salt of a fatty acid having from 6 to 14 carbon atoms and a crystallisation inhibitor which prevents or reduces crystal formation from said solution, wherein the crystallisation inhibitor is a branched chain fatty acid having from 8 to 22 carbon atoms present.

In a second aspect of the present invention, there is provided a non-therapeutic method for reducing body odour comprising the topical application of a deodorant composition according to the first aspect of the invention.

In a third aspect of the present invention, there is provided the non-therapeutic use of a composition according to the first aspect of the invention for reducing body odour on the surface of the human body.

In a fourth aspect of the present invention, there is provided a method of stabilising [anhydrous] [ethanolic] solutions of a zinc dicarboxylate salt of a fatty acid having from 6 to 14 carbon atoms, said method comprising the inclusion of a crystallisation inhibitor for the zinc dicarboxylate salt, wherein the crystallisation inhibitor is a branched chain fatty acid having from 8 to 22 carbon atoms present.

### Detailed Description

Herein, features expressed as "preferred" with regard to a particular aspect of the invention should be understood to be preferred with regard to each aspect of the invention (likewise, features expressed as "more preferred" or "most preferred").

Herein, preferred features of the invention are particularly preferred when used in combination with other preferred features.

Herein, "ambient conditions" refers to about 20°C and 1 atmosphere pressure, unless otherwise indicated. Observations and requirement referred to herein are under ambient conditions unless otherwise indicated.

Herein, all numbers, amounts and ratios may optionally be understood to be modified by the word "about", unless otherwise indicated.

Herein, all percentages, ratios and amounts are by weight, unless otherwise indicated.

Herein, all percentages are by weight of the total composition, excluding any volatile propellant that may be present therein.

Herein, the word "comprising" is intended to mean "including" but not necessarily "consisting of", i.e., it is non-exhaustive.

Herein, "free water" includes all water in the composition with the exception of water of crystallisation associated with any solid components present therein.

Herein, references to "zinc dicarboxylate salts" are to zinc dicarboxylate salts as described in the first aspect of the invention.

The topical application of a deodorant composition as specified and as referred to in the above paragraph is preferably to the underarm regions of the human body. These are the regions most commonly in need of malodour reduction.

The zinc dicarboxylate salt used in the present invention is a salt of a fatty acid having from 6 to 14 carbon atoms. Such salts are relative hydrophobic in nature, enhancing the ease with which they can be formulated with other hydrophobic components typically used in cosmetic compositions, but causing some issues in ethanolic solutions (*vide infra*).

The zinc dicarboxylate salts used in the present invention are not as hydrophobic and of such high melting as zinc ricinoleate, which is particularly difficult to formulate because of its hydrophobicity and high melting point.

Preferred zinc dicarboxylate salts used in the present invention are liquids at 20°C and atmospheric pressure, i.e. they have a melting point of less than 20°C. This aids their formulation into cosmetic compositions.

Preferred zinc dicarboxylate salts used in the present invention are salts of branched chain fatty acids, particularly when saturated and especially when having from 8 to 12 carbon atoms.

Particularly preferred zinc dicarboxylate salts are salts of an α-branched fatty acid, i.e. a fatty acid having a branch, typically an alkyl branch, on the carbon atom adjacent to the carboxylic acid group (the "α-carbon", designated as 2- in IUPAC nomenclature). Such fatty acids are preferably of from 8 to 12 carbon atoms and are independently or in combination preferred to have three alkyl substituents at the α-carbon.

An especially preferred zinc dicarboxylate used in the present invention is zinc neodecanoate, which is a salt of zinc and neodecanoic acid having the formula [C₉H₂₀-CO.O]₂Zn.

Neodecanoic acid is a mixture of carboxylic acids. Components of the mixture are "trialkyl acetic acids", having three alkyl substituents at the α-carbon, including 2,2,3,5-tetramethylhexanoic acid; 2,4-dimethyl-2-isopropylpentanoic acid; 2,5-dimethyl-2-ethylhexanoic acid; 2,2-dimethyloctanoic acid; and 2,2-diethylhexanoic acid.

In compositions according to the invention, the zinc dicarboxylate is preferably present at from 0.1 to 20% and more preferably at from 1 to 10%, ignoring any volatile propellant that may be present in the composition.

The zinc dicarboxylate is present in ethanolic solution, by which is meant a solution in which ethanol is the predominant solvent, comprising greater than 50% of the composition, excluding any volatile propellant that may be present therein.

The ethanol content in compositions of the invention is preferably at least 70% and more preferably at least 90%, again excluding any volatile propellant that may be present therein.

The composition is preferably a homogeneous solution.

Preferred compositions contain less than 1.3% by weight of free water, more preferably they contain less than 0.8% of free water, and most preferably they contain less than 0.25% of free water. These water levels aid the stability of the ethanolic solutions with regard to crystal formation.

Particular problems have surprisingly been observed with ethanolic solutions of zinc dicarboxylate salts having a low level of water (less than 0.25%). First, we have observed that such solutions generate crystalline deposits, and this can be detrimental both for sensory reasons and because of the possibility of valve blockage when the composition is applied as an aerosol product.

A second problem is observed during factory processing. When a low water content ethanolic solution of zinc dicarboxylate has been used in a vessel to manufacture a deodorant composition, the vessel subsequently needs to be cleaned, typically using the same low water content ethanolic solvent used in the manufacture. Surprisingly, we have found that this leads to crystalline deposits and these are an issue when it comes to subsequent re-use of the processing vessel.

A solution to both of the above problems has been found in the use of crystallisation inhibitors as described herein.

Crystallisation inhibitors as used herein prevent or at least reduce crystal formation from ethanolic solutions of zinc dicarboxylate solutions, particularly such solutions having a low water content (less than 0.25%). Further, such crystallisation inhibitors prevent or at least reduce crystal formation during the cleaning with low water content ethanol of vessels used in the manufacture of deodorant compositions comprising a low water content ethanolic solution of a zinc dicarboxylate salt.

The crystallisation inhibitors used herein are able to prevent or delay nucleation and/or reduce the growth rate of one or more crystal faces. The need for such materials in compositions of the invention is particularly surprising when the zinc dicarboxylate is a liquid, such as when the zinc dicarboxylate is zinc neodecanoate.

When the zinc dicarboxylate is zinc neodecanoate, it is understood that the crystals that form in the absence of a crystallisation inhibitor are a modified form of zinc neodecanoate that is able to form crystals and has a melting point of above ambient temperature.

The present inventors have devised a simple test for preferred crystallisation inhibitors using zinc neodecanoate in ethanolic solution. In a first step of the test, a solution of zinc neodecanoate (2% by weight) is made in ethanol having a water content of less 0.1% by weight. In a second step of the test, this solution is diluted 100x (100 times) with the same ethanol used in the first step. Preferred crystallisation inhibitors pass the test by showing no crystal formation in the resulting solution after a period of at least 12 hours.

Crystallisation inhibitors are selected from branched chain fatty acids having from 8 to 22 carbon atoms. Other materials have been found to be less effective (*vide infra*).

Particularly preferred crystallisation inhibitors are α-branched chain fatty acid and in some preferred embodiments they have three alkyl substituents at the α-carbon.

In some preferred embodiments the crystallisation inhibitor is selected from the group consisting of 2,2-di-(n-propyl)acetic acid, neodecanoic acid, 2-butyloctanoic acid and isostearic acid.

In especially preferred embodiments the crystallisation inhibitor is neodecanoic acid.

The crystallisation inhibitor is present in amount that is preferably from 0.05 to 3% of the total composition, excluding any volatile propellant that may be present therein.

The weight ratio of the crystallisation inhibitor to the zinc dicarboxylate salt is preferably at least 1: 33 and more preferably at least 1: 25. The preferred upper level of crystallisation inhibitor may be determined by factors including rheological stability and sometimes malodour issues, but its weight ratio to the zinc dicarboxylate is typically up to 2: 1 and preferably up to 1: 1, especially in combination with the preferred lower levels indicated in this paragraph.

In preferred embodiments, the composition comprises a fragrance. This may give rise to a deodorant composition having a triple mode of action: reducing microbial numbers, entrapment of malodours and malodour masking.

Encapsulated fragrances may also be advantageously employed, particularly those that are friable, i.e. released by the application of shear forces, such as those that may be encountered in the underarm regions of the human body.

In compositions comprising a fragrance, the total amount of fragrance is preferably up to 8% and more preferably up to 5% and the total amount of fragrance is preferably from 0.5%, more preferably from 1.0%, ignoring any volatile propellant present in the composition. In a particularly preferred embodiment, the total amount of fragrance is from 1 to 5%, again ignoring any volatile propellant that may be present in the composition.

An oil is a preferred component of compositions aid the present invention. Herein, "oils" are water-immiscible liquids having a melting point above 20°C.

Preferred oils are hydrocarbon oils, in particular saturated hydrocarbon oils having from 10 to 14 carbon atoms.

Other preferred oils are ester or ether oils. Examples of suitable ester oils include oils containing one or two alkyl groups of 12 to 24 carbon atoms length, including isopropyl myristate, isopropyl palmitate and myristyl palmitate. Other examples are liquid triglyceride oils, such as sunflower seed oil. Yet other suitable ester oils are alkyl or aryl benzoates such C₁₂₋₁₅ alkyl benzoate.

Examples of suitable ether oils include polypropylene glycol alkyl ethers such as PPG-14-butylether and PPG-15-stearyl ether.

When an aerosol spray compositions is used in the delivery of the present invention, a volatile propellant is typically employed. Herein, a volatile propellant is one having a boiling point of less than 10°C and one that may preferably be liquified at pressures within a typical aerosol spray can, i.e. at from 1.5 to 3 bar.

Typical volatile propellants are hydrocarbon or halogenated hydrocarbon gas (particularly fluorinated hydrocarbons such as 1,1-difluoroethane and/or 1-trifluoro-2-fluoroethane). It is preferred that the volatile propellant comprises liquefied hydrocarbon gases, especially C₃ to C₆ hydrocarbons, including propane, isopropane, butane, isobutane, and mixtures of two or more thereof.

A preferred component of some compositions used in the present invention is an antioxidant, such as BHT (butylated hydroxytoluene) or dilauryl thiodipropionate, typically used at a level of from 0.01 to 5%, excluding any volatile propellant that may be present.

### Examples

The following materials were amongst those tested in this study:
The zinc neodecanoate used was a mixture with isopropyl myristate (4: 1 by weight) and a small amount of fragrance.

Ethanol (minimum DEB 99.5% w/w, denatured with 0.0010 Bitrex and 0.1% t-butyl alcohol), ex Alcodis. The ethanol used had a water content of less than 0.1%.
2-Butyloctanoic acid (Isocarb 12, ex Sasol).
Isostearyl alcohol (Prisorine 3515, ex Croda).
Octyldodecanol (Eutanol G, ex BASF).

The following studies were performed at ambient temperature. A 2.2% solution of zinc neodecanoate in ethanol was prepared and aliquots of this solution were added to sample tubes. Amounts of the test substances as indicated in Table 1 were then added to the sample tubes. The amounts indicated were the total concentration of the test substance in the mixture.

After formation of the mixtures, these were diluted 100x with ethanol and left overnight for at least 12 hours. Some mixtures generated crystals, indicating that the test substance was unsuitable as a crystallisation inhibitors at the level added. Other mixtures did not generate crystals on dilution, indicating that the test substance was suitable as a crystallisation inhibitors at the level added. This test mimics the effect of washing out vessels previously used with the pre-dilution concentration of zinc neodecanoate with low water content ethanol. It also represents an "accelerated storage test" for the mixtures, samples failing the test (by generating crystals) also being expected to form crystals at the pre-dilution concentration.

**Table 1**

| Example | Test substance | Level added (%) | Result |
|---|---|---|---|
| 1 | 2,2-di-(n-propyl)acetic acid | 1.0 | No crystals formed |
| 2 | 2,4-dimethyl-2-pentenoic acid | 1.0 | No crystals formed |
| 3 | Neodecanoic acid | 0.5 | No crystals formed |
| 4 | | 0.08 | No crystals formed |
| 5 | | 0.04 | Crystals formed |
| 6 | | 0.02 | Crystals formed |
| 7 | | 0.01 | Crystals formed |
| 8 | | 0.005 | Crystals formed |
| 9 | None | -- | Crystals formed |
| 10 | 2-butyloctanoic acid | 1.0 | No crystals formed |
| 11 | Isostearic acid | 0.5 | No crystals formed |
| 12 | | 2.0 | No crystals formed |
| 13 | Undecane | 0.5 | Crystals formed |
| 14 | | 2.0 | Crystals formed |
| 15 | Decane | 0.5 | Crystals formed |
| 16 | | 2.0 | Crystals formed |
| 17 | Isoamyl laurate | 0.5 | Crystals formed |
| 18 | | 2.0 | Crystals formed |
| 19 | Octyldodecanol | 0.5 | Crystals formed |
| 20 | | 2.0 | Crystals formed |
| 21 | Isostearyl alcohol | 0.5 | Crystals formed |
| 22 | | 2.0 | Crystals formed |
| 23 | Decanoic acid | 0.5 | Crystals formed |
| 24 | | 2.0 | Crystals formed |
| 25 | Lauric acid | 0.5 | Crystals formed |
| 26 | | 2.0 | Crystals formed |

It may be seen that test substances that are branched chain fatty acids having from 8 to 22 carbon atoms prevent the formation of crystals whereas other test substances were less successful in this regard.

For neodecanoic acid, it seemed that an inclusion level of greater than 0.04% was required for success.

## Claims

1. A deodorant composition which is an ethanolic solution comprising a zinc dicarboxylate salt of a fatty acid having from 6 to 14 carbon atoms and a crystallisation inhibitor which prevents or reduces crystal formation from said solution, wherein the crystallisation inhibitor is a branched chain fatty acid having from 8 to 22 carbon atoms present.

2. A deodorant composition according to claim 1, comprising less than 0.25% by weight of free water, based upon the weight of the composition excluding any volatile propellant that may be present therein.

3. A deodorant composition according to claim 1 or claim 2, wherein the crystallisation inhibitor passes a test whereby a solution of zinc neodecanoate (2% by weight) is made in ethanol having a water content of less than 0.1% by weight, this solution is subsequently diluted 100x with the same ethanol used to form the initial solution, and the crystallisation inhibitor passes the test by showing no crystal formation in the diluted solution after a period of at least 12 hours.

4. A deodorant composition according to any one of the preceding claims, wherein the crystallisation inhibitor is selected from the group consisting of 2,2-di-(n-propyl)acetic acid, neodecanoic acid, 2-butyloctanoic acid and isostearic acid.

5. A deodorant composition according to claim 4, wherein the crystallisation inhibitor has three alkyl substituents at the α-carbon.

6. A deodorant composition according to claim 5, wherein the crystallisation inhibitor is neodecanoic acid.

7. A deodorant composition according to any one of the preceding claims, wherein the weight ratio crystallisation inhibitor to the zinc dicarboxylate salt is from 1: 33 to 1: 1.

8. A deodorant composition according to any one of the preceding claims in the form of an aerosol composition.

9. A deodorant composition according to claim 8 comprising a volatile propellant.

10. A deodorant composition according to any one of the preceding claims, wherein the ethanolic solution is homogeneous.

11. A deodorant composition according to any one of the preceding claims, wherein the zinc dicarboxylate salt is zinc neodecanoate.

12. A non-therapeutic method for reducing body odour comprising the topical application of a deodorant composition according to any of the preceding claims.

13. The non-therapeutic use of a composition according to any of claims 1 to 11 for reducing body odour on the surface of the human body.

## Patentansprüche

1. Deodorant-Zusammensetzung, die eine ethanolische Lösung ist, umfassend ein Zinkdicarboxylatsalz einer Fettsäure mit 6 bis 14 Kohlenstoffatomen und einen Kristallisationsinhibitor, der die Kristallbildung aus dieser Lösung verhindert oder verringert, wobei der Kristallisationsinhibitor eine verzweigtkettige Fettsäure mit 8 bis 22 Kohlenstoffatomen ist.

2. Deodorant-Zusammensetzung nach Anspruch 1, die weniger als 0,25 Gew.-% freies Wasser umfasst, bezogen auf das Gewicht der Zusammensetzung, wobei ein gegebenenfalls darin enthaltenes flüchtiges Treibmittel außer Betracht bleibt.

3. Deodorant-Zusammensetzung nach Anspruch 1 oder Anspruch 2, wobei der Kristallisationsinhibitor einen Test besteht, bei dem eine Lösung von Zinkneodecanoat (2 Gew.-%) in Ethanol mit einem Wassergehalt von weniger als 0,1 Gew.-% hergestellt wird, wobei diese Lösung anschließend mit demselben Ethanol, das zur Herstellung der Ausgangslösung verwendet wurde, 100-fach verdünnt wird und der Kristallisationsinhibitor den Test besteht, indem er nach einer Zeitspanne von mindestens 12 Stunden keine Kristallbildung in der verdünnten Lösung zeigt.

4. Deodorant-Zusammensetzung nach irgendeinem der vorhergehenden Ansprüche, wobei der Kristallisationsinhibitor aus der Gruppe ausgewählt ist, die aus 2,2-Di-(n-propyl)essigsäure, Neodecansäure, 2-Butyloctansäure und Isostearinsäure besteht.

5. Deodorant-Zusammensetzung nach Anspruch 4, wobei der Kristallisationsinhibitor drei Alkylsubstituenten am α-Kohlenstoff aufweist.

6. Deodorant-Zusammensetzung nach Anspruch 5, wobei der Kristallisationsinhibitor Neodecansäure ist.

7. Deodorant-Zusammensetzung nach irgendeinem der vorhergehenden Ansprüche, wobei das Gewichtsverhältnis von Kristallisationsinhibitor zu Zinkdicarboxylatsalz zwischen 1:33 und 1:1 liegt.

8. Deodorant-Zusammensetzung nach irgendeinem der vorhergehenden Ansprüche in Form einer Aerosolzusammensetzung.

9. Deodorant-Zusammensetzung nach Anspruch 8, die ein flüchtiges Treibmittel enthält.

10. Deodorant-Zusammensetzung nach irgendeinem der vorhergehenden Ansprüche, wobei die ethanolische Lösung homogen ist.

11. Deodorant-Zusammensetzung nach irgendeinem der vorhergehenden Ansprüche, wobei das Zinkdicarboxylatsalz Zinkneodecanoat ist.

12. Nichttherapeutisches Verfahren zur Verringerung von Körpergeruch, umfassend die topische Anwendung einer Deodorant-Zusammensetzung nach einem der vorhergehenden Ansprüche.

13. Nichttherapeutische Verwendung einer Zusammensetzung nach einem der Ansprüche 1 bis 11 zur Verringerung des Körpergeruchs auf der Oberfläche des menschlichen Körpers.

## Revendications

1. Composition déodorante qui est une solution éthanolique comprenant un sel dicarboxylate de zinc d'un acide gras ayant de 6 à 14 atomes de carbone et un inhibiteur de cristallisation qui empêche ou réduit la formation de cristaux à partir de ladite solution, dans laquelle l'inhibiteur de cristallisation est un acide gras à chaîne ramifiée ayant de 8 à 22 atomes de carbone présents.

2. Composition déodorante selon la revendication 1, comprenant moins de 0,25 % en poids d'eau libre, par rapport au poids de la composition, à l'exclusion de tout propulseur volatil qui peut être présent dans celle-ci.

3. Composition déodorante selon la revendication 1 ou la revendication 2, dans laquelle l'inhibiteur de cristallisation réussit un test dans lequel une solution de néodécanoate de zinc (2 % en poids) est produite dans de l'éthanol ayant une teneur en eau inférieure à 0,1 % en poids, cette solution est ensuite diluée 100 fois avec le même éthanol utilisé pour former la solution initiale, et l'inhibiteur de cristallisation réussit le test en ne présentant aucune formation de cristaux dans la solution diluée après une période d'au moins 12 heures.

4. Composition déodorante selon l'une quelconque des revendications précédentes, dans laquelle l'inhibiteur de cristallisation est choisi dans le groupe consistant en l'acide 2,2-di-(n-propyl)acétique, l'acide néodécanoïque, l'acide 2-butyloctanoïque et l'acide isostéarique.

5. Composition déodorante selon la revendication 4, dans laquelle l'inhibiteur de cristallisation a trois substituants alkyle sur le carbone α.

6. Composition déodorante selon la revendication 5, dans laquelle l'inhibiteur de cristallisation est l'acide néodécanoïque.

7. Composition déodorante selon l'une quelconque des revendications précédentes, dans laquelle le rapport pondéral de l'inhibiteur de cristallisation au sel dicarboxylate de zinc est de 1:33 à 1:1.

8. Composition déodorante selon l'une quelconque des revendications précédentes sous forme d'une composition d'aérosol.

9. Composition déodorante selon la revendication 8 comprenant un propulseur volatil.

10. Composition déodorante selon l'une quelconque des revendications précédentes, dans laquelle la solution éthanolique est homogène.

11. Composition déodorante selon l'une quelconque des revendications précédentes, dans laquelle le sel dicarboxylate de zinc est le néodécanoate de zinc.

12. Procédé non thérapeutique pour réduire les odeurs corporelles comprenant l'application topique d'une composition déodorante selon l'une quelconque des revendications précédentes.

13. Utilisation non thérapeutique d'une composition selon l'une quelconque des revendications 1 à 11 pour réduire les odeurs corporelles sur la surface du corps humain.
